# EUROPÄISCHE PATENTSCHRIFT

(11) **EP 2 321 263 B1**
(45) Veröffentlichungstag und Bekanntmachung des Hinweises auf die Patenterteilung: **07.06.2017**
(21) Anmeldenummer: 09781839.7
(22) Anmeldetag: 14.08.2009
(51) Int. Cl.: C07C 249/04, C07C 251/44, B01J 8/20

(54) **REAKTOR MIT TITANSILIKAT-REZYKLIERUNG**
REACTOR WITH TITANIUM SILICATE RECYCLING
RÉACTEUR COMPRENANT UN SYSTÈME DE RECYCLAGE DU SILICATE DE TITANE

(30) Priorität: 08.09.2008 DE 102008041870
(43) Veröffentlichungstag der Anmeldung: 18.05.2011
(73) Patentinhaber: Evonik Degussa GmbH, 45128 Essen (DE)
(72) Erfinder: BAUMGARTEN, Götz, 45721 Haltern am See (DE); ROOS, Martin, 45721 Haltern am See (DE); SCHÄFLEIN, Stephan, 13509 Berlin (DE); AUGENSTEIN, Rolf, 45770 Marl (DE)
(86) Internationale Anmeldenummer: PCT/EP2009/060536
(87) Internationale Veröffentlichungsnummer: WO 2010/026031

(56) Entgegenhaltungen:
- EP-A- 1 468 986
- EP-A- 1 674 450
- CN-Y- 200 951 385
- DE-A1- 3 245 318

## Beschreibung

Die vorliegende Erfindung betrifft eine Anlage zur Durchführung chemischer Reaktionen mit mindestens einem Reaktor, dessen Reaktionsraum Wasserstoffperoxid und Titansilikalit enthält. Bekannt ist eine solche Anlage aus der EP 1 316 545 B1. Ferner betrifft die Erfindung eine besondere Verwendung dieser Anlage und ein dank der Anlage ermöglichtes Verfahren zur Durchführung einer Reaktion.

Zahlreiche industriell durchgeführte chemische Reaktionen finden in Gegenwart von Titansilikalit statt, welches dabei als Katalysator dient. Als Beispiel sei die Ammoximierung genannt, also das Herstellen von Oximen aus Ketonen oder Aldehyden. Hinsichtlich dieser Reaktion wird auf die EP 1 316 545 B1 ebenso wie auf die EP 1 191 017 B1 verwiesen, auf deren Offenbarungsgehalt hier vollständig Bezug genommen wird.

Bei dem genannten Titansilikalit handelt es sich um einen Katalysator auf Basis von Titan, Silizium und Sauerstoff; vorzugsweise in zeolithischer Struktur. Derartige Katalysatoren sind unter der Produktbezeichung "TS-1" bei der Evonik Degussa GmbH erhältlich.

Unter den Reaktionsbedingungen der Ammoximierung ist der in dem Titansilikalit enthaltene SiO₂-Anteil in dem System löslich. Deswegen wird der Katalysator durch ungesättigte SiO₂-Ströme um SiO₂ abgereichert, sodass im Reaktionsraum unlösliche, inerte, nanoskalige TiO₂-Partikel entstehen. Diese keramischen Kleinstpartikel werden dispergiert in Wasser - welches unter Anderem durch Reaktion aus H₂O₂ entsteht und aus der Reaktion abgezogen werden muss - aus dem Reaktor ausgetragen und belasten aufgrund ihrer geringen Größe nachgeschaltete Anlagen der Abwasseraufbereitung. Eine wirksame Abfilterung der Katalysatorrückstände ist zwingend erforderlich.

Katalysatorlösungen werden häufig mit klassischen Trennapparaten wie beispielsweise Filtern, Zentrifugen oder Hydrozyklonen abgeschieden. Im vorliegenden Fall führen solche Verfahren bedingt durch den nanoskaligen Anteil aktiven Katalysators jedoch nicht zu akzeptablen Katalysatorverlusten im Retentat bzw. im Klarlauf. Hinzu kommt der diskontinuierliche Charakter dieser Verfahren, die nur mit entsprechendem Aufwand in eine kontinuierliche Reaktionsführung eingegliedert werden können.

Aus der DE 32 45 318 C3 ist es bekannt, Katalysatordispensionen mit dem Querstromfiltrations-Verfahren abzutrennen und kontinuierlich in die Reaktion zurückzuführen. In Laborversuchen wurde jedoch festgestellt, dass sich die Querstrom-Filtration mit keramischen Ultrafiltrationsmembranen nicht ohne Weiteres mit einer Ammoximierung kombinieren lässt, da die dabei entstehenden nanoskaligen TiO₂-Partikel nach kurzer Zeit Deckschichten auf der Ultrafiltrationsmembran bilden und zu einer irreversiblen Verblockung des Filters führen:

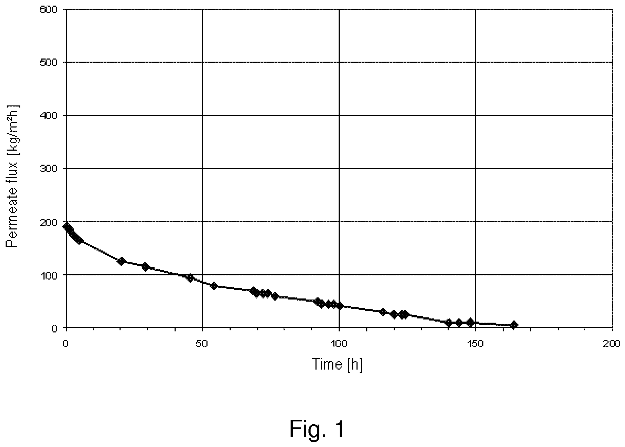

Figur 1 zeigt den Permeatflussabfall einer unter ammoniakalischen Bedingungen (5 %ige NH₃-Lösung) kontinuierlich mit einer 1.5 %igen TS-1-Katalysatorslurry-Dispersion beschickten Keramik-Membran mit einer Trenngrenze von 0.05 µm. Bereits nach 150 Stunden war der Permeatfluss irreversibel auf weniger als 25 kg/m²h abgefallen. Dabei waren übliche Betriebsbedingungen für die Membran gewählt worden. Der Transmembrandruck lag bei 0.5 bar und die Überstromgeschwindigkeit bei etwa 4 m/s. Die Permeatausbeute lag unter 10 % bezogen auf die Einspeisung. Rasterelektronenaufnahmen zeigen, dass unter den Reaktionsbedingungen SiO₂ aus dem Katalysator herausgelöst wird dabei gebildetes, nanoskaliges TiO₂ die Membran zusetzt.

Die US 7 408 080 B2 schlägt vor, die Auflösung des Titansilikalits bei der Ammoximierung durch Zugabe einer flüssigen Siliziumquelle zu unterbinden. Versuche zeigen jedoch, dass diese Maßnahme bei ungünstigen Reaktionsbedingungen zu einer schlagartigen Verblockung der Membran führen kann. Dies ist darauf zurückzuführen, dass die Zugabe der flüssigen Siliziumquelle das Reaktionsgemisch übersättigt, was wiederum eine plötzliche Kristallisation (Ausfallen) der Siliziumoxide hervorruft. Die ausgefallenen Kristallpartikel setzten die Filtermembran abrupt zu.

EP-A-1468986 offenbart ein diskontinuierliches Verfahren zur Herstellung von Cyclohexanonoxim durch Ammoximierung, wobei der Katalysator durch Filtration rezykliert und zur Reaktionsmischung zugegeben wird.

In Hinblick auf diesen Stand der Technik liegt der vorliegenden Erfindung die Aufgabe zugrunde, eine Anlage der eingangs genannten Gattung so weiterzubilden, dass eine kontinuierliche Abtrennung und Rückführung des aktiven Katalysators in den Reaktionsraum mit einer langen Filterstandzeit möglich ist.

Gelöst wird diese Aufgabe durch eine Anlage für die Durchführung chemischer Reaktionen, die mindestens einem Reaktor aufweist, dessen Reaktionsraum Wasserstoffperoxid und Titansilikalit enthält,
- wobei der Reaktionsraum eine feste Siliziumquelle enthält,
- wobei die Anlage eine Wasserabzugsleitung umfasst, die dafür eingerichtet ist, Wasser nebst darin gelöster und/oder dispergierter Komponenten aus dem Reaktionsraum abzuleiten,
- wobei die Wasserabzugsleitung zu einem die in dem Wasser gelösten und/oder dispergierten Komponenten abscheidendes Filter führt,
- wobei die Anlage eine Rückführleitung umfasst, welche dafür eingerichtet ist, mittels des Filters abgeschiedene Komponenten in den Reaktionsraum zurückzuführen, und
- dass dem Reaktor ein flüssig/flüssig-Abscheider zugeordnet ist, welcher das aus dem Reaktionsraum abzuleitende Wasser nebst darin gelöster
- und/oder dispergierter Komponenten von übrigen im Reaktionsraum befindlichen, flüssigen Komponenten abscheidet.

Der vorliegenden Erfindung liegt die Erkenntnis zu Grunde, dass es mit Hilfe der Dosierung eines festen Silizium-Opfersystems gelingt, den Auflösungsprozess des Katalysators auf dem Niveau der nanoskaligen Katalysatorspartikeln wirksam zu stoppen, das Entstehen von inertem TiO₂ zu vermeiden, die Bildung von irreversiblen Deckschichten auf Filtrationsmembranen wirksam vorzubeugen und damit letztendlich die Filterstandzeit zu erhöhen.

Die Zugabe des festen Si-Opfersystems ermöglicht die effektive Durchführung der Reaktion unter Anwesenheit von Titansilikalit und Wasserstoffperoxid auf der erfindungsgemäßen Anlage, bei der die Abscheidung des Katalysators und/oder seiner Rückstände mittels eines Filter erfolgt, welches mit der Wasserabzugsleitung bzw. mit der Rücklaufleitung mit dem Reaktionsraum verbunden ist und so eine Rezyklierung des mit dem überschüssigen Reaktionswasser ausgeschwemmten Titansilikalits erlaubt. Bei dem genannten Wasser handelt es sich in der Regel nicht um reines H₂O, sondern um eine wässrige Phase der Reaktion, die gelöste, dispergierte oder insbesondere suspendierte Komponenten enthält. Der Begriff "Wasserabzugsleitung" versteht sich deswegen allgemein als beherrschte Stoffströmung der wässrigen Reaktionsphase in Richtung des Filters. Es ist nämlich nicht zwingend erforderlich, die Wasserabzugsleitung als Rohrleitung auszuführen: Stattdessen kann eine erfindungsgemäße Anlage innerhalb ihrer Organe ein definiertes Strömungsprofil ausbilden, welche einen Strömungspfad umfasst, entlang dessen der Massestrom des Reaktionswassers verläuft. Dies soll später näher erläutert werden.

Hinsichtlich der Verwendung der festen Siliziumquelle unterscheidet sich die Erfindung von dem in US 7 408 080 B2 gelehrten, flüssigen Opfersystem. Das Problem des plötzlichen Ausfallens von Silizium-Kristallen aus übersättigter Lösung tritt bei der Verwendung des Feststoffes nicht auf, da sich dieser stets nur zu einem solchen Anteil im System löst, wie es dessen gegenwärtige Löslichkeit zulässt. Es findet quasi eine hochgenaue, selbstregulierende Dosierung des Siliziums statt, die mit apparativem Aufbau zur Dosierung der Zugabe einer flüssigen Siliziumquelle nicht erreichbar ist: Selbst wenn flüssiges Silizium nur tropfenweise zugegeben würde, entstünde in der unmittelbaren Umgebung des Tropfens im Reaktionsgemisch eine örtliche Übersättigung mit Silizium, die eine Fällreaktion verursachen kann. Die Zugabe des Feststoffes senkt den apparativen Dosierungs-Aufwand bei Verbesserung der Dosier-Genauigkeit deutlich.

Als Si-Opfersystem eignet sich eine Vielzahl von siliziumhaltigen Feststoffen. Besonders bevorzugt ist die Verwendung von gefällter Kieselsäure, da diese - verglichen mit dem Katalysator - preiswert ist und deswegen geopfert werden kann.

Der Reaktionsraum, in welchem die Reaktion durchgeführt wird, muss sich nicht zwangsläufig auf einen einzelnen Reaktor erstrecken. Es ist denkbar die Anlage mit einer Reaktorkaskade auszurüsten, also mit einer Mehrzahl von hintereinander geschalteten Einzelreaktoren, wobei jeder Folgereaktor aus dem Überlauf seines Vorgängers gespeist wird. Die Wasserabzugsleitung für den ersten Reaktor versteht sich dann als ein Strömungspfad entlang der Überläufe durch die Folgereaktoren hindurch bis zu dem Filter.

Soweit die Reaktion mehrphasig ist, erfordert das Abziehen des Reaktionswassers regelmäßig eine vorherige Trennung der wässrigen Phase. Die Phasentrennung kann im Reaktionsraum selbst stattfinden, beispielsweise durch Abschalten des Rührwerks, sofern sich die Phasen daraufhin voneinander absetzen. Ist dies nicht der Fall oder wird eine kontinuierliche Verfahrensführung angestrebt, kann dem Reaktor ein flüssig/flüssig-Abscheider zugeordnet werden, welcher das aus dem Reaktionsraum abzuleitende Wasser nebst darin gelöster und/oder dispergierter Komponenten von übrigen im Reaktionsraum enthaltenen, flüssigen Komponenten abscheidet. Die Reaktion und die Phasentrennung erfolgt dann in unterschiedlichen Organen der Anlage. Die Wasserabzugsleitung versteht sich in diesem Zusammenhang als ein Strömungspfad aus dem Reaktor in den Abscheider und von dort zum Filter.

So die Phasentrennung im Reaktor selbst erfolgt, kann das Filter vorteilhafterweise in den Reaktor eingegliedert werden. Eine Rohrleitung für den Wasserabzug ist dann teilweise entbehrlich, stattdessen würde ein Abschnitt als Strömungspfad durch den Reaktionsraum selbst verlaufen, wodurch Kosten gespart werden. Das Filter kann dann beispielhaft als Teil der Reaktorwand ausgeführt sein.

Eine besonders vorteilhafte Ausführungsform der Erfindung sieht vor, das Filter als Filterrohr auszuführen, welches das in den meisten Reaktoren vorhandene Rührwerk mantelartig umgibt. Der Fluss durch den Filter wird dann von dem Rührwerk aufrecht erhalten. Eine solche Konstellation erlaubt somit das Einsparen einer zusätzlichen Pumpe.

Vorzugsweise handelt es sich bei dem Filter um ein Membran-Filter mit keramischer Membran, da eine solche Abtrenneinheit beständig ist gegen wässrige, ammoniakalische, H₂O₂-haltige Lösung bei etwa 85 bis 95 °C, wie sie bei der Ammoximierung aus dem Reaktionsraum mittels der Wasserabzugsleitung abgezogen und durch das Filter geführt wird.

In besonderer Weise eigenen sich Querstrom-Membranfilter als Filter, da sie zum einen die Katalysatoren sicher abzuscheiden vermögen und zum anderen eine hohe kontinuierliche Filterleistung erreichen und sich so in einen fließenden Industrieprozess integrieren lassen.

Vorzugsweise wird die erfindungsgemäße Anlage zur Durchführung einer insbesondere zweiphasigen Ammoximierung verwendet. Die erfindungsgemäß erzielten Vorteile lassen sich aber auch bei anderen Reaktionen nutzen, deren System Titansilikalit gewöhnlich löst.

Die Durchführung einer unter Anwesenheit von Titansilikalit durchgeführten Reaktion geschieht auf der erfindungsgemäßen Anlage wie folgt:
- Wasser mit darin gelöstem und/oder dispergiertem Titansilikalit wird aus der Reaktion abgezogen,
- das abgezogene Titansilikalit wird über das Filter aus dem Wasser abgetrennt und in die Reaktion zurückgeführt,
- der Reaktion wird eine feste Siliziumquelle zugegeben.

Bevorzugt handelt es sich bei der Siliziumquelle um gefällte Kieselsäure, einem Feststoff, der vergleichsweise preiswert ist.

In einer besonders bevorzugten Ausführungsform der Erfindung beträgt die Konzentration der Siliziumquelle mindestens der Konzentration des Katalysators: Das Verhältnis der Masse der Siliziumquelle zu der Masse des Titansilikalits betrage in der Reaktion 1:1. Beste Ergebnisse werden erreicht, wenn das Verhältnis etwa 3:1 beträgt, also die dreifache Konzentration an Silizium gegenüber dem Katalysator.

Das Abziehen des Wassers und das Rückführen des abgetrennten Katalysators kann periodisch erfolgen, nämlich nach vorübergehendem Abschalten der Rühranlage des Reaktors, sodass sich räumlich getrennte Phasen im Reaktor herausbilden, die das Abziehen der wässrigen Phase vereinfachen. Allerdings ist eine kontinuierliche Verfahrensführung wirtschaftlicher; diese gelingt mit Hilfe eines flüssig/flüssig-Abscheiders, welcher die wässrige Phase der Reaktion außerhalb des Reaktors abscheidet.

Das erfindungsgemäße Verfahren eignet sich hervorragend zur Durchführung alkalischer Reaktionen, vor allem zur Durchführung einer zweiphasigen Ammoximierung.

Die vorliegende Erfindung soll nun anhand eines bevorzugten Ausführungsbeispiels unter Zuhilfenahme der beiliegenden Abbildungen näher erläutert werden. Hierfür zeigen:
- Figur 1:: Permeatflussabfall bei der TS-1-Katalysatorslurry-Abtrennung mit Keramik-Membran ohne Zugabe von Kieselsäure (Stand der Technik);
- Figur 2:: wie Figur 1, jedoch mit Zugabe von Kieselsäure (erfindungsgemäß);
- Figur 3:: Schaltbild einer Anlage mit Einzelreaktor und separatem Filter (erste Ausführungsform);
- Figur 4:: Schaltbild einer Anlage mit Einzelreaktor und in dessen Wand eingebetteten Filter (zweite Ausführungsform);
- Figur 5:: Schaltbild einer Anlage mit Einzelreaktor und Filterrohr (dritte Ausführungsform);
- Figur 6:: Schaltbild einer Anlage mit Reaktorkaskade und separatem Abscheider (vierte Ausführungsform);
- Figur 7:: Veränderung des Umsatzes von CDON in der Anlage mit und ohne Zugabe von Kieselsäure.

Der zu Figur 1 gehörige Versuch wurde oben zur Erläuterung des der Erfindung zugrunde liegenden Problems beschrieben.

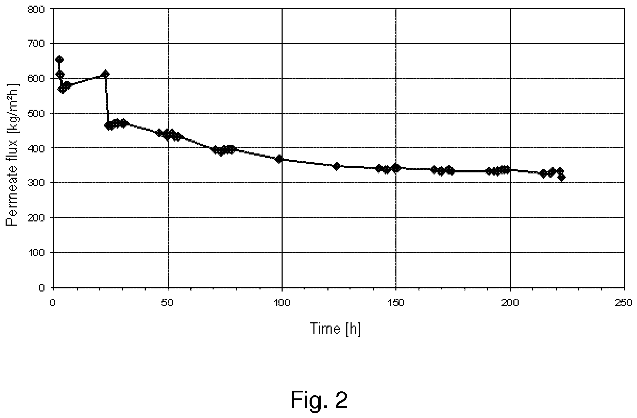

Figur 2 zeigt demgegenüber als Maß für die Filtrationsleistung der Membran den Verlauf des Permeatflusses nach Zugabe von 1.5 % des Feststoffes "Sipernat 320" (eine der Evonik Degussa GmbH erhältliche gefällte Kieselsäure) zur 1.5 %igen TS-1 (Titansilikalit)-Dispersion unter ansonsten gleichen Bedingungen wie bei dem Figur 1 zugrunde liegendem Versuch. Zu erkennen ist, dass die erfindungsgemäße Verfahrensführung eine asymptotische Annäherung an einen nahezu konstanten Sockel-Permeatfluss bewirkt, währenddessen ohne Sipernat-Zugabe der Fluss durch die sich zusetzende Membran rasch versiegt (vgl. Figur 1).

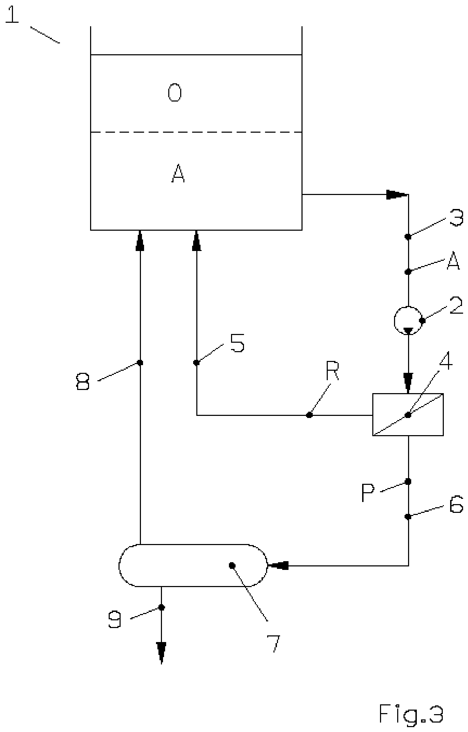

Die verlässliche Abscheideleistung des Filters erlaubt den Aufbau einer Anlage zur kontinuierlichen Durchführung einer zweiphasigen Ammoximierung. Gleichwohl sind auch periodisch betriebene Anlagen Gegenstand der Erfindung. Eine erste periodisch betriebene Anlage ist in Figur 3 symbolisch dargestellt. Sie umfasst einen Reaktor 1, in welchem - wie in EP 1 316 545 B1 gelehrt - eine zweiphasige Ammoximierung durchgeführt wird. Bei diesem Verfahren bilden sich bei Reaktionsbedingungen (Druck: 4 bis 6 bar; Temperatur: 95 bis 105 °C) im Reaktionsraum zwei flüssige Phasen aus, eine organische Phase O und eine wässrige Phase A. Die organische Phase O wird gebildet aus der Ausgangskomponente Cyclodecanon (kurz: CDON) und der Zielkomponente Cyclododecanonoxim (kurz: 0-xim) beides gelöst in dem organischen Lösungsmittel Isopropylcyclohexan (genannt: Hydrocumol). Die wässrige Phase A enthält gelöst Ammoniak (NH₃), Wasserstoffperoxid (H₂O₂) sowie Ammoniumsalze (NH₄⁺x⁻). Suspendiert sind in der flüssigen Phase Titansilikalit (TS-1) und mit der dreifachen Konzentration gefällte Kieselsäure (Sipernat). Der pH-Wert der wässrigen Phase beträgt etwa 9 bis 11. Aufgrund ihres hohen Anteils an dispergierten Anteilen hat die wässrige Phase eine breiige Konsistenz, man spricht von einem Slurry. Als Phasenvermittler zwischen W und A können Tenside wie "Marlon PS 30" der Sasol Germany GmbH vorgesehen sein. Die beiden Phasen O und A sind im normalen Reaktionsbetrieb nicht - wie in Fig. 3 dargestellt - räumlich voneinander getrennt, sondern werden mit Hilfe eines in Fig.3 nicht dargestellten Rührwerks miteinander vermischt.

Um das bei der Reaktion entstehende Wasser aus dem Reaktor zu entfernen wird das Rührwerk einstweilen abgeschaltet, sodass sich die Phasen wie dargestellt aufeinander absetzen. Sodann wird Slurry mit Hilfe einer Pumpe 2 entlang einer Wasserabzugsleitung 3 aus dem Reaktorraum abgepumpt und zu einem Querstrom-Membranfilter 4 gefördert. Die Temperatur der wässrigen Phase A in der Wasserabzugsleitung 3 beträgt vor dem Filter 4 etwa 85 °C bis 95 °C. Die keramische Filtermembran des Querstrom-Filters 4 scheidet die mit dem Wasser ausgeschwemmten Feststoffe TS-1 und Sipernat aus der wässrigen Phase ab. Das Retentat R enthaltend TS-1 und Sipernat wird als konzentrierter Slurry entlang einer Rückführleitung 5 in den Reaktor 1 rezykliert. Das Permeat P enthaltend Wasser, Ammoniak und Ammoniumsalze gelangt weiter über eine Permeatleitung 6 zu einem Destillator 7. Dieser gewinnt in Wasser konzentriert gelösten Ammoniak zurück; der letztere gelangt über eine Ammoniakrückleitung 8 erneut in die Reaktion. Das Restwasser mit den darin gelösten Ammoniumsalzen verlässt durch Abwasserleitung 9 die Anlage. Nach abgeschlossenem Rückgewinnungszyklus wird das Rührwerk wieder eingeschaltet und die Reaktion mit vermischten Phasen fortgeführt. Der Wechsel der Betriebszustände erfolgt periodisch. Um den Prozess kontinuierlich zu fahren, müssten zwei Reaktoren parallel geschaltet werden, von denen jeweils einer sich stets im Reaktionsbetrieb befindet, währenddessen der andere rezykliert.

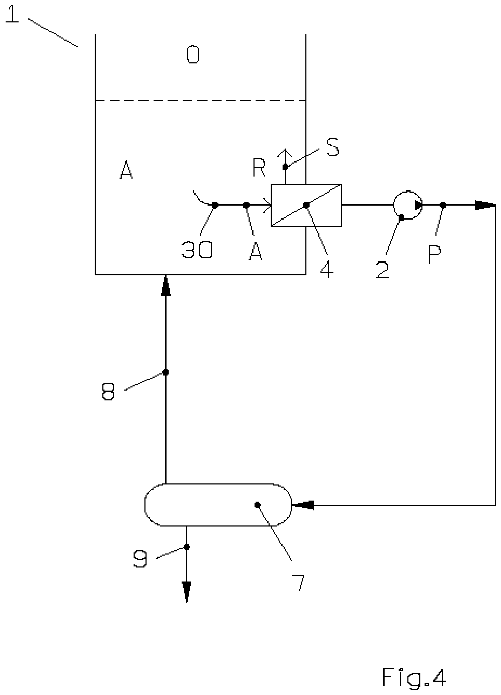

Einen höheren Intergrationsgrad erreicht die in Figur 4 ausschnittsweise dargestellte Anlage. Das Filter 4 ist hier unmittelbar in die Wand des Reaktors 1 eingebettet, was Rohrleitung einspart. Ein (sehr kurzer) Abschnitt der Wasserabzugsleitung 3 verläuft als Strömungspfad 30 innerhalb des Teils des Reaktorraums, den die wässrige Phase A einnimmt. Ein Vorteil dieser Gestaltung besteht darin, dass in Ermangelung eines Rohrleitungsabschnittes zwischen Reaktionsraum und Filter praktisch kein Druckverlust auftritt; der Reaktionsdruck (4 bis 6 bar) lastet unmittelbar auf der Membran des Filters 4, was im Vergleich zu der in Figur 3 gezeigten Ausführungsform den Permeatfluss steigert. Rückführleitung 5 für Retentat R kann dann kurz ausfallen.

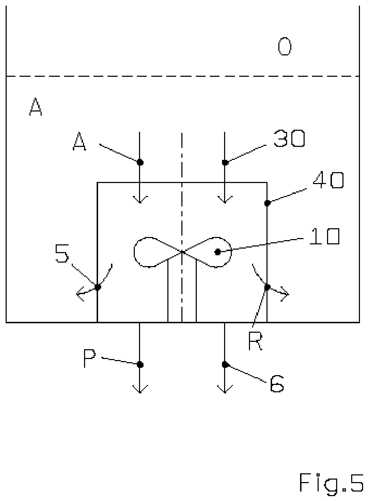

Bei der in Figur 5 gezeigten Ausführungsform kann gegenüber der in Figur 4 gezeigten Anlage sogar die Pumpe eingespart werden: Dies gelingt dadurch, dass das Filter als ein das im Reaktor 1 ohnehin erforderliche Rührwerk 10 umgebendes, zylindrisches Filterrohr 40 ausgeführt ist. Das koaxial zum Filterrohr 40 rotierende Rührwerk 10 erzeugt innerhalb des Reaktorraums ein Strömungsprofil, welches entlang eines Strömungspfades 30 einen Massestrom des Reaktionswassers durch die Membran des Filterrohres 40 umfasst. Der Massenstrom des Retentats R tritt durch das Filterrohr 40 direkt in den Reaktionsraum zurück. Die Rückführleitung 5 entspricht damit einem unkanalisierten Strömungspfad.

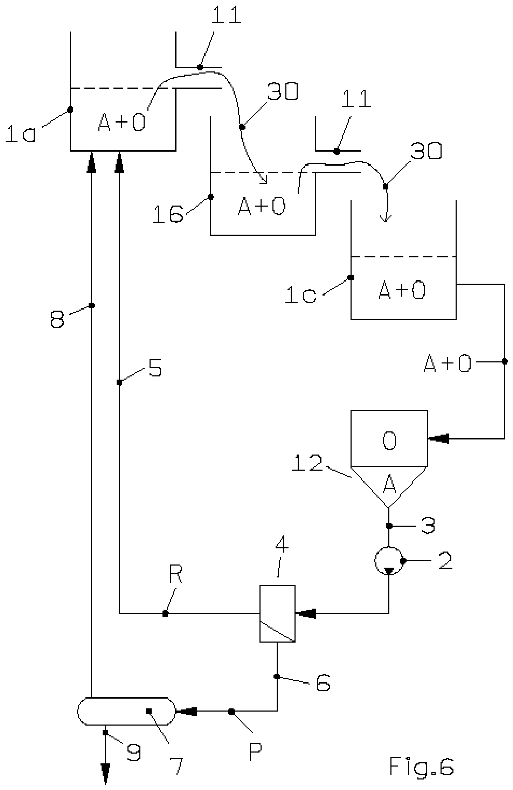

Die vorstehend erläuterten Ausführungsbeispiele zeigen eine saubere Trennung zwischen organischer Phase O und wässriger Phase A innerhalb des Reaktionsraumes. Dies ist anzustrebsam, da das Filter 4 prinzipbedingt nicht zur Phasentrennung geeignet ist. Soweit die Reaktion nicht zwischen räumlich getrennten Phasen stattfindet, ist eine Phasentrennung erforderlich, etwa durch periodisches Ausschalten des Rührwerkes 10. Diese diskontinuierliche Verfahrensführung ist bei der zweiphasigen Ammoximierung unwirtschaftlich; es ist ein kontinuierlicher Betrieb anzustreben, welchen die in Figur 6 gezeigte Anlage gestattet:
Der Reaktionsraum dieser Anlage ist auf drei Reaktoren 1 a, 1 b, 1 c verteilt, die hintereinander zu einer Kaskade verschaltet sind. Der sekundäre und der tertiäre Reaktor 1 b und 1 c werden jeweils aus dem Überlauf 11 des Vorgängers 1 a bzw. 1 b gespeist. Das Reaktortemperatur steigt innerhalb der Kaskade 1a, 1b, 1c von Reaktor zu Reaktor an, um die Löslichkeit des Oxims in dem Hydrocumol zu steigern. Die Phasen A und O sind innerhalb der Reaktoren 1a, 1b, 1c nicht räumlich voneinander getrennt, sondern stets ineinander vermengt. Um dennoch Wasser abziehen zu können, ist dem letzten Reaktor 1 c ein flüssig/flüssig-Abscheider 12 zugeordnet, welcher die Phasentrennung ausserhalb des Reaktionsraums vornimmt. Der Abscheider 12 wirkt stömungsberuhigend und weist in seinem unteren Bereich einen konischen Abschnitt auf, in welchem sich die schwerere wässrige Phase A absetzt. Von der Spitze des konischen Abschnittes aus ist die Wasserabzugsleitung 3 als Rohrleitung ausgeführt; ausgehend von der in der Kaskade räumlich verteilten Phase A bis zur genannten Spitze versteht sich die Wasserabzugsleitung als Strömungspfad 30 durch die Reaktoren 1a, 1 b, 1 c, entlang deren Überläufe 11 bis in den Abscheider 12. Rohrleitung 3 führt dann das Reaktionswasser A weiter über die Pumpe 2 zum Filter 4, wo das Retanat R für die Rezyklierung des Katalysators über die Rückführleitung 5 abgefiltert wird. Im Übrigen kann das Abfiltern des Retanats kann auch vor der Phasentrennung erfolgen, ein flüssig/flüssig-Abscheider ist dann in der Rückführleitung anzuordnen.

Die soeben geschilderte Anlage erlaubt dank Zugabe der gefällten Kieselsäure eine die kontinuierliche Durchführung einer zweiphasigen Ammoximierung mit einem nahezu konstantem Umsatz von CDON:
So wurde der Einfluss der Sipernat-Zugabe auf die Umsetzung von CDON mit NH₃ (25 %ig) und H₂O₂ (50 %ig) an TS-1-Katalysatorslurry in einem System Wasser/Hydrocumol zum Oxim untersucht. Die Umsetzung des Speisestroms von 1.82 kg/h CDON erfolgte bei einem gestuft ansteigenden Temperaturprofil von 90 bis 105 °C in einer Kaskade aus drei Rührkesselreaktoren mit jeweils 10 I Reaktorrauminhalt, einer anschließenden Phasenseparation zur Produktabtrennung in einem separaten Abscheider und einer anschließenden Membranfiltration zur Abtrennung der wässrigen Phase unter Rückführung des Katalysatorslurrys in die Reaktorkaskade. Im System wurde im erfindungsgemäßen Beispiel eine TS1-Katalysatormasse von 1 kg neben 2 kg gefällter Kieselsäure vom Typ Sipernat vorgelegt.

Im Gegenbeispiel wurde das Sipernat weggelassen. Aus dem System wurden mit dem Ammoniak- und dem Reaktionswasser bei einem Massenstrom von 2.56 kg/h jeweils 900 ppm SiO₂ aus dem System ausgetragen. Durch diesen Austrag war der Katalysator ohne Opfersystem (Gegenbeispiel) nach 700 h soweit aufgelöst, dass der CDON-Umsatz von urspünglich 98-99 % auf 40 % gesunken ist. Mit der Zugabe einer Menge gefällter Kieselsäure, die größer ist als die aus dem System ausgetragene SiO₂-Menge (erfindungsgemäßes Beispiel) bleibt der Katalysator TS-1 erhalten.

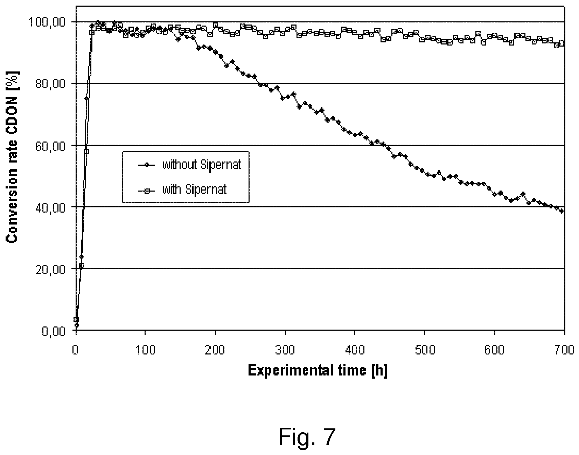

Figur 7 verdeutlicht die Abnahme des Umsatzes von CDON ohne Zugabe eines Si-Opfersystems im Vergleich zu einem erfindungemäßen System, dem das Si-Opfersystem Sipernat zugegeben wurde.

## Patentansprüche

1. Anlage für die Durchführung chemischer Reaktionen, mit mindestens einem Reaktor (1), dessen Reaktionsraum Wasserstoffperoxid und Titansilikalit enthält,
**dadurch gekennzeichnet,**
a) **dass** der Reaktionsraum eine feste Siliziumquelle enthält,
b) **dass** die Anlage eine Wasserabzugsleitung (3) umfasst, die dafür eingerichtet ist, Wasser nebst darin gelöster und/oder dispergierter Komponenten aus dem Reaktionsraum abzuleiten,
c) **dass** die Wasserabzugsleitung (3) zu einem die in dem Wasser gelösten und/oder dispergierten Komponenten abscheidendes Filter (4) führt,
d) **dass** die Anlage eine Rückführleitung (5) umfasst, welche dafür eingerichtet ist, mittels des Filters (4) abgeschiedene Komponenten in den Reaktionsraum zurückzuführen und
e) **dass** dem Reaktor (1) ein flüssig/flüssig-Abscheider (12) zugeordnet ist, welcher das aus dem Reaktionsraum abzuleitende Wasser nebst darin gelöster und/oder dispergierter Komponenten von übrigen im Reaktionsraum befindlichen, flüssigen Komponenten abscheidet.

2. Anlage nach Anspruch 1,
**dadurch gekennzeichnet,**
**dass** es sich bei der festen Siliziumquelle um gefällte Kieselsäure handelt.

3. Anlage nach Anspruch 1 oder 2,
**gekennzeichnet durch**
eine Vielzahl von zu einer Kaskade hintereinander geschalteten Reaktoren (1a, 1b, 1c).

4. Anlage nach einem der Ansprüche 1 bis 3,
**dadurch gekennzeichnet,**
**dass** das Filter (4) in den Reaktor (1) eingegliedert ist, wobei es sich bei der Wasserabzugsleitung zumindest abschnittsweise um einen Strömungspfad (30) innerhalb eines im Reaktor (1) gebildeten Strömungsprofils handelt.

5. Anlage nach Anspruch 4,
**dadurch gekennzeichnet,**
**dass** das Filter (4) in die Reaktorwand eingegliedert ist.

6. Anlage nach Anspruch 4,
wobei der Reaktor (1) mit einem Rührwerk (10) versehen ist,
**dadurch gekennzeichnet,**
**dass** das Filter (4) als ein das Rührwerk (10) umgebendes Filterrohr (40) ausgeführt ist.

7. Anlage nach einem der Ansprüche 1 bis 6,
**dadurch gekennzeichnet,**
**dass** es sich bei dem Filter (4) um ein Membran-Filter mit keramischer Membran handelt.

8. Anlage nach Anspruch 7,
**dadurch gekennzeichnet,**
**dass** es sich bei dem Filter (4) um ein Querstrom-Filter handelt.

9. Verwendung einer Anlage nach einem der Ansprüche 1 bis 8 zur Durchführung einer Ammoximierung, insbesondere einer zweiphasigen Ammoximierung.

10. Verfahren zur Durchführung einer unter Anwesenheit von Titansilikalit durchgeführten Reaktion,
**dadurch gekennzeichnet,**
**dass** in kontinuierlicher Verfahrensführung Wasser mit darin gelöstem und/oder dispergiertem Titansilikalit aus der Reaktion abgezogen wird, dass das abgezogene Titansilikalit über ein Filter aus dem Wasser abgetrennt und in die Reaktion zurückgeführt wird, und dass der Reaktion eine feste Siliziumquelle zugegeben wird.

11. Verfahren nach Anspruch 10,
**dadurch gekennzeichnet,**
**dass** es sich bei der festen Siliziumquelle um gefällte Kieselsäure handet.

12. Verfahren nach Anspruch 10 oder 11,
**dadurch gekennzeichnet,**
**dass** das Verhältnis der Masse der Siliziumquelle zu der Masse des Titansilikalits 1:1 oder größer, vorzugsweise etwa 3:1 beträgt.

13. Verfahren nach einem der Ansprüche 10 bis 12,
**dadurch gekennzeichnet,**
**dass** das Abziehen des Wassers und das Rückführen des abgetrennten Titansilikalits kontinuierlich erfolgt.

14. Verfahren nach einem der Ansprüche 10 bis 12,
**dadurch gekennzeichnet,**
**dass** die Reaktion unter alkalischen Bedingungen durchgeführt wird.

15. Verfahren nach Anspruch 14,
**dadurch gekennzeichnet,**
**dass** es sich bei der Reaktion um eine Ammoximierung, insbesondere um eine zweiphasige Ammoximierung handelt.

16. Verwendung einer festen Siliciumquelle als Opfersystem zum Stoppen des Auflösungsprozesses eines Titansilikalit-Katalysators.

17. Verwendung nach Anspruch 16,
**dadurch gekennzeichnet,**
**dass** es sich bei der festen Siliziumquelle um gefällte Kieselsäure handelt.

## Claims

1. Plant for carrying out chemical reactions, having at least one reactor (1), the reaction space of which contains hydrogen peroxide and titanium silicalite, **characterized**
a) **in that** the reaction space contains a solid silicon source,
b) **in that** the plant comprises a water takeoff line (3) which is set up for drawing off water in addition to components which are dissolved and/or dispersed therein from the reaction space,
c) **in that** the water takeoff line (3) leads to a filter (4) which separates off the components which are dissolved and/or dispersed in the water,
d) **in that** the plant has a return line (5) which is set up for recirculating to the reaction space components which are separated off by means of the filter (4) and
e) **in that** a liquid/liquid separator (12) is assigned to the reactor (1), which separator separates the water which is to be drawn off from the reaction space in addition to components which are dissolved and/or dispersed therein from remaining liquid components which are situated in the reaction space.

2. Plant according to Claim 1, **characterized in that** the solid silicon source is precipitated silicic acid.

3. Plant according to Claim 1 or 2, **characterized by** a multiplicity of reactors connected one after the other to form a cascade (1a, 1b, 1c).

4. Plant according to any one of Claims 1 to 3, **characterized in that** the filter (4) is incorporated into the reactor (1), wherein the water takeoff line at least in sections is a flow path (30) within a flow profile formed in the reactor (1).

5. Plant according to Claim 4, **characterized in that** the filter (4) is incorporated into the reactor wall.

6. Plant according to Claim 4, wherein the reactor (1) is provided with an agitator (10), **characterized in that** the filter (4) is constructed as a filter tube (40) surrounding the agitator (10).

7. Plant according to any one of Claims 1 to 6, **characterized in that** the filter (4) is a membrane filter having a ceramic membrane.

8. Plant according to Claim 7, **characterized in that** the filter (4) is a cross flow filter.

9. Use of a plant according to any one of Claims 1 to 8 for carrying out an ammoximation, in particular a two-phase ammoximation.

10. Process for carrying out a reaction carried out in the presence of titanium silicalite, **characterized in that**, in a continuous process, water having titanium silicalite dissolved and/or dispersed therein is taken off from the reaction, **in that** the titanium silicalite which is taken off is separated off from the water via a filter and recirculated to the reaction, and **in that** a solid silicon source is added to the reaction.

11. Process according to Claim 10, **characterized in that** the solid silicon source is precipitated silicic acid.

12. Process according to Claim 10 or 11, **characterized in that** the ratio of the mass of the silicon source to the mass of the titanium silicalite is 1:1 or greater, preferably about 3:1.

13. Process according to any one of Claims 10 to 12, **characterized in that** the water is taken off, and the titanium silicalite separated off is recirculated continuously.

14. Process according to any one of Claims 10 to 12, **characterized in that** the reaction is carried out under alkaline conditions.

15. Process according to Claim 14, **characterized in that** the reaction is an ammoximation, in particular a two-phase ammoximation.

16. Use of a solid silicon source as sacrificial system for stopping the dissolution process of a titanium silicalite catalyst.

17. Use according to Claim 16, **characterized in that** the solid silicon source is precipitated silicic acid.

## Revendications

1. Unité pour la réalisation de réactions chimiques, comprenant au moins un réacteur (1), dont la chambre de réaction contient du peroxyde d'hydrogène et du silicalite de titane,
**caractérisée en ce que**
a) la chambre de réaction contient une source de silicium solide,
b) l'unité comprend une conduite de soutirage d'eau (3), qui est conçue pour décharger de la chambre de réaction de l'eau conjointement avec des composants dissous et/ou dispersés dans celle-ci,
c) la conduite de soutirage d'eau (3) conduit vers un filtre (4) qui sépare les composants dissous et/ou dispersés dans l'eau,
d) l'unité comprend une conduite de recyclage (5), qui est conçue pour recycler les composants séparés au moyen du filtre (4) dans la chambre de réaction, et
e) un séparateur liquide/liquide (12) est attribué au réacteur (1), qui sépare l'eau à décharger de la chambre de réaction conjointement avec les composants dissous et/ou dispersés dans celle-ci des autres composants liquides se trouvant dans la chambre de réaction.

2. Unité selon la revendication 1, **caractérisée en ce que** la source de silicium solide est de la silice précipitée.

3. Unité selon la revendication 1 ou 2, **caractérisée par** une pluralité de réacteurs raccordés en série en cascade (1a, 1b, 1c).

4. Unité selon l'une quelconque des revendications 1 à 3, **caractérisée en ce que** le filtre (4) est intégré dans le réacteur (1), la conduite de soutirage d'eau correspondant au moins en partie à un chemin d'écoulement (30) dans un profil d'écoulement formé dans le réacteur (1).

5. Unité selon la revendication 4, **caractérisée en ce que** le filtre (4) est intégré dans la paroi du réacteur.

6. Unité selon la revendication 4, dans laquelle le réacteur (1) est muni d'un agitateur (10), **caractérisée en ce que** le filtre (4) est configuré sous la forme d'un tube de filtre (40) entourant l'agitateur (10).

7. Unité selon l'une quelconque des revendications 1 à 6, **caractérisée en ce que** le filtre (4) est un filtre à membrane comprenant une membrane céramique.

8. Unité selon la revendication 7, **caractérisée en ce que** le filtre (4) est un filtre à courant transversal.

9. Utilisation d'une unité selon l'une quelconque des revendications 1 à 8 pour la réalisation d'une ammoximation, notamment d'une ammoximation en deux phases.

10. Procédé de réalisation d'une réaction réalisée en présence de silicalite de titane, **caractérisé en ce que**, pendant une exploitation continue du procédé, de l'eau est soutirée de la réaction conjointement avec du silicalite de titane dissous et/ou dispersé dans celle-ci, **en ce que** le silicalite de titane extrait est séparé de l'eau par un filtre et recyclé dans la réaction, et **en ce qu'**une source de silicium solide est ajoutée à la réaction.

11. Procédé selon la revendication 10, **caractérisé en ce que** la source de silicium solide est de la silice précipitée.

12. Procédé selon la revendication 10 ou 11, **caractérisé en ce que** le rapport entre la masse de la source de silicium et la masse du silicalite de titane est de 1:1 ou plus, de préférence d'environ 3:1.

13. Procédé selon l'une quelconque des revendications 10 à 12, **caractérisé en ce que** le soutirage de l'eau et le recyclage du silicalite de titane séparé ont lieu en continu.

14. Procédé selon l'une quelconque des revendications 10 à 12, **caractérisé en ce que** la réaction est réalisée dans des conditions alcalines.

15. Procédé selon la revendication 14, **caractérisé en ce que** la réaction est une ammoximation, notamment une ammoximation en deux phases.

16. Utilisation d'une source de silicium solide en tant que système sacrificiel pour stopper le processus de dissolution d'un catalyseur à base de silicalite de titane.

17. Utilisation selon la revendication 16, **caractérisée en ce que** la source de silicium solide est de la silice précipitée.
